# EUROPEAN PATENT APPLICATION

(11) **EP 4 342 498 A1**
(43) Date of publication of application: **27.03.2024**
(21) Application number: 22807561.0
(22) Date of filing: 13.05.2022
(51) Int. Cl.: A61K 48/00, A61P 3/06, A61P 3/10, A61P 9/10, A61P 27/12, A61P 43/00, A61P 17/00, A61P 17/14, C12N 15/113, C12N 15/86, A61K 35/76, A61K 31/7088

(54) **ANTISENSE OLIGOMER**

(30) Priority: 13.05.2021 JP 2021081389
(71) Applicant: National University Corporation Chiba University, Chiba-shi, Chiba 263-8522 (JP)
(72) Inventor: YOKOTE, Koutaro, Chiba-shi, Chiba 2608670 (JP); OUCHI, Yasuo, Chiba-shi, Chiba 2608670 (JP); KATO, Hisaya, Chiba-shi, Chiba 2608677 (JP); ETO, Koji, Chiba-shi, Chiba 2608675 (JP); MAEZAWA, Yoshiro, Chiba-shi, Chiba 2608670 (JP)
(74) Representative: Hoffmann Eitle
(86) International application number: PCT/JP2022/020224
(87) International publication number: WO 2022/239863

(57) **Abstract**

The present invention is to provide an antisense oligomer or a pharmaceutically acceptable salt thereof capable of treating Werner syndrome without direct repair of a mutated gene. The present invention provides an antisense oligomer or a pharmaceutically acceptable salt thereof which consists of a base sequence complementary to the base sequence of the following (i) or (ii): (i) a contiguous base sequence of 15 or more bases in the base sequence shown in SEQ ID NO: 1; or (ii) a contiguous base sequence of 15 or more bases in the base sequence shown in SEQ ID NO: 1, having deletion, substitution, or insertion of one or more bases; and which is capable of causing skipping of the 27th exon in a human WRN gene.

## Description

### Technical Field

The present invention relates to an antisense oligomer or a pharmaceutically acceptable salt thereof, a viral vector, and a pharmaceutical composition.

### Background Art

Werner syndrome is a form of progeria in which aging rapidly accelerates after puberty and causes symptoms such as progeroid hair changes (e.g., gray hair, baldness), ocular cataracts (bilateral), skin atrophy/skin sclerosis (e.g., corns, callus), refractory ulceration, soft-tissue calcifications (e.g., Achilles tendons), disorder of glucose/lipid metabolism, premature arteriosclerosis (e.g., angina pectoris, myocardial infarctions). While the median lifespan has been improving thanks to recent studies, many patients suffer from serious complications of lower limb amputation associated with refractory skin ulceration, malignant tumors, and diabetes.

Approximately 80% of the patients with Werner syndrome reported globally are Japanese.

Werner syndrome is a rare autosomal recessive hereditary disease, and the cause thereof is considered to be an abnormality of the human WRN gene, which encodes DNA helicase (see, e.g., Non-Patent Literatures 1 and 2).

In recent years, to some hereditary diseases such as Duchenne muscular dystrophy and spinal muscular atrophy, therapeutic agents using exon skipping have been proposed. For example, Patent Literature 1 discloses a composition for use in producing skipping of exon 44 in the processing of human dystrophin pre-processed mRNA, comprising a specific antisense compound, as a means for treating dystrophy.

### Citation List

### Patent Literature

Patent Literature 1: WO 2010/048586

### Non Patent Literature

Non Patent Literature 1: Yokote K et al., Hum Mutat. 2017 January; 38(1):7-15.
Non Patent Literature 2: Kato H, et al., Stem Cell Research. Vol. 53, May 2021, 102360

### Summary of Invention

### Problems to Be Solved by The Invention

Werner syndrome is an intractable disease, for which there is no possible treatment measure capable of completely curing the disease other than repairing of a mutation of the WRN gene in a patient. Nowadays, development of technology for repairing a genetic mutation, such as genome editing technique, has been receiving expectations. However, repairing a mutation of the WRN gene in the whole body or a lesion site is difficult to be clinically applied with the current technique.

To diseases other than Werner syndrome, a therapeutic agent using exon skipping by an antisense oligomer, which does not directly repair a mutated gene, has been proposed. However, such a proposal for Werner syndrome has not been reported.

An object of the present invention is to provide a means for treating Werner syndrome without direct repair of the mutated gene.

### Solution to Problem

The present inventors have found that an antisense oligomer consisting of a specific base sequence is capable of causing skipping of the 27th exon in a human WRN gene, and treating Werner syndrome without direct repair of a mutated gene.

The present invention encompasses the following embodiments.
[1] An antisense oligomer or a pharmaceutically acceptable salt thereof which consists of a base sequence complementary to the base sequence of the following (i) or (ii):
   (i) a contiguous base sequence of 15 or more bases in a base sequence shown in SEQ ID NO: 1; or
   (ii) a contiguous base sequence of 15 or more bases in a base sequence shown in SEQ ID NO: 1, having deletion, substitution, or insertion of one or more bases;
   and which is capable of causing skipping of the 27th exon in a human WRN gene.
[2] The antisense oligomer or the pharmaceutically acceptable salt thereof according to [1], the contiguous base sequence in the base sequence (i) or the base sequence (ii) has a length of 20 bases or more and 40 bases or less.
[3] The antisense oligomer or the pharmaceutically acceptable salt thereof according to [1] or [2], in which the contiguous base sequence in the base sequence (i) or the base sequence (ii) comprises at least one of the base sequence shown in SEQ ID NO: 2 and the base sequence shown in SEQ ID NO: 3.
[4] The antisense oligomer or the pharmaceutically acceptable salt thereof according to [3], in which the contiguous base sequence in the base sequence (i) or the base sequence (ii) comprises at least one of the base sequence shown in SEQ ID NO: 4 and the base sequence shown in SEQ ID NO: 5.
[5] The antisense oligomer or the pharmaceutically acceptable salt thereof according to any one of [1] to [4], in which the contiguous base sequence in the base sequence (i) or the base sequence (ii) consists of the base sequence shown in any of SEQ ID NOs: 6 to 15.
[6] The antisense oligomer or the pharmaceutically acceptable salt thereof according to any one of [1] to [5], in which the antisense oligomer is an oligonucleotide, a morpholino oligomer, a peptide nucleic acid (PNA) oligomer, or a glycol nucleic acid (GNA) oligomer.
[7] The antisense oligomer or the pharmaceutically acceptable salt thereof according to any one of [1] to [5], in which the antisense oligomer is a morpholino oligomer.
[8] The antisense oligomer or the pharmaceutically acceptable salt thereof according to [7], in which the morpholino oligomer is a phosphorodiamidate morpholino oligomer.
[9] The antisense oligomer or the pharmaceutically acceptable salt thereof according to [6], in which the oligonucleotide is an oligonucleotide comprising one or more selected from the group consisting of bridged nucleic acid (BNA) nucleotides.
[10] The antisense oligomer or the pharmaceutically acceptable salt thereof according to any one of [1] to [9], in which at least one of the 5' end and the 3' end is modified.
[11] A viral vector which expresses RNA capable of causing skipping of the 27th exon in a human WRN gene, said RNA consists of a base sequence complementary to the base sequence of the following (i) or (ii):
   (i) a contiguous base sequence of 15 or more bases in the base sequence shown in SEQ ID NO: 1; or
   (ii) a contiguous base sequence of 15 or more bases in the base sequence shown in SEQ ID NO: 1, having deletion, substitution, or insertion of one or more bases.
[12] The viral vector according to [11], in which the contiguous base sequence in the base sequence (i) or the base sequence (ii) comprises the base sequence shown in SEQ ID NO: 16.
[13] A pharmaceutical composition for the treatment of Werner syndrome, comprising the antisense oligomer or the pharmaceutically acceptable salt thereof according to any one of [1] to [10], or the viral vector according to [11] or [12] as an active ingredient.
[14] The pharmaceutical composition for the treatment of Werner syndrome according to [13], comprising two or more of the antisense oligomers or the pharmaceutically acceptable salts thereof as an active ingredient.
[15] A pharmaceutical composition for inducing skipping of the 27th exon in a human WRN gene, comprising the antisense oligomer or the pharmaceutically acceptable salt thereof according to any one of [1] to [10], or the viral vector according to [11] or [12] as an active ingredient.

### Advantageous Effects of Invention

The present invention provides an antisense oligomer or a pharmaceutically acceptable salt thereof capable of causing skipping of the 27th exon in a human WRN gene, and treating Werner syndrome without direct repair of a mutated gene.

### Brief Description of Drawings

[Figure 1] Figure 1 shows results of RT-PCR analysis.
[Figure 2] Figure 2 shows quantitative results of exon skipping efficiency.
[Figure 3] Figure 3 shows results of Western blotting analysis.
[Figure 4] Figure 4 shows results of cell growth curve analysis.
[Figure 5] Figure 5 shows results of PDL analysis.
[Figure 6] Figure 6 shows results of cell morphology observation.
[Figure 7] Figure 7 shows results of SA-β-Gal staining analysis.
[Figure 8] Figure 8 shows results of SA-β-Gal staining quantitative analysis.
[Figure 9] Figure 9 shows results of telomere length analysis.
[Figure 10] Figure 10 shows results of gene expression level analysis.
[Figure 11] Figure 11 shows results of RT-PCR analysis.
[Figure 12] Figure 12 shows quantitative results of exon skipping efficiency.

### Description of Embodiments

### [Antisense Oligomer]

The antisense oligomer or the pharmaceutically acceptable salt thereof of the present invention is an antisense oligomer or a pharmaceutically acceptable salt thereof which consists of a base sequence complementary to the following base sequence (i) or (ii):
(i) a contiguous base sequence of 15 or more bases in the base sequence shown in SEQ ID NO: 1; or
(ii) a contiguous base sequence of 15 or more bases in the base sequence shown in SEQ ID NO: 1, having deletion, substitution, or insertion of one or more bases;
and which is capable of causing skipping of the 27th exon in a human WRN gene.

The antisense oligomer or the pharmaceutically acceptable salt thereof of the present invention is capable of causing skipping of the 27th exon (hereinafter, also referred to as "exon 27") in a human WRN gene.

Hereinafter, the "antisense oligomer or the pharmaceutically acceptable salt thereof" may be described simply as "antisense oligomer".

### <Human WRN Gene>

Human WRN genes are genes which encode DNA helicase, and a gene dysfunction due to mutation of the genes is considered to be the cause of Werner syndrome.

Genome base sequences in the human WRN gene region, base sequences of cDNA, and amino acid sequences of a protein to be encoded are known. The base sequences in the human WRN gene obtained from the human genome project as a reference have been registered in Genbank provided by National Center for Biotechnology Information (NCBI), under the following accession number (when a plurality of revisions are registered, it is understood to refer to the latest revision):
Human WRN gene: NM_000553

### <Skipping of the 27th Exon>

A major mature transcription product (mature mRNA) of the human WRN gene is made up of 35 exons.

70.7% of the patients with Werner syndrome reported in Japan have c.3139-1G>C mutation in the human WRN gene, that is, a mutation from a guanine (G) base to cytosine (C) in an intron just one base before the 3139th base counted from a translation start codon in a cDNA base sequence. The mutation is a mutation in a splicing acceptor sequence of the 26th exon (hereinafter, also referred to as "exon 26") in the WRN gene, and induces skipping of exon 26 to generate a translation stop codon due to a frameshift in exon 27. Consequently, the major functional domains of the WRN protein comprising a nuclear localization signal present at the C-terminal is deleted, the function of the WRN gene is largely damaged and hence, it is considered to be the cause of Werner syndrome.

The antisense oligomer of the present invention targets mature mRNA in the human WRN gene and is capable of causing skipping of exon 27. As a result, when skipping of exon 26 occurs due to c.3139-1G>C mutation, mature mRNA in which the 25th exon and the 28th exon are linked to each other is produced.

In the present invention, the mature mRNA in which exon 27 as well as exon 26 were removed by skipping has a further frameshift due to the removal of exon 27, and encodes a mutant-type WRN protein capable of protein translation up to a wild-type translation stop codon. The mutant-type WRN protein encodes a protein consisting of 1375 amino acid residues comprising the nuclear localization signal present at the C-terminal, and has a deletion of 3.9% (57 amino acid residues) with respect to a wild-type WRN protein consisting of 1432 amino acid residues. Surprisingly, however, the mutant-type WRN protein exhibits recovery of at least some functions from the gene dysfunction due to c.3139-1G>C mutation, as shown in Examples later.

The occurrence of skipping of exon 27 in the human WRN gene can be confirmed in mRNA of the human WRN gene by, for example, as shown in Examples later, using a primer pair set upstream and downstream of the region of skipped exon 27, performing RT-PCR amplification in a region comprising before and after exon 27, and performing PCR amplification or sequence analysis on the resulted PCR amplified product.

### <Base Sequence>

The antisense oligomer of the present invention consists of a base sequence complementary to the base sequence of the following (i) or (ii):
(i) a contiguous base sequence of 15 or more bases in the base sequence shown in SEQ ID NO: 1; or
(ii) a contiguous base sequence of 15 or more bases in the base sequence shown in SEQ ID NO: 1, having deletion, substitution, or insertion of one or more bases.

### (Base Length)

The base sequence of the antisense oligomer of the present invention has a length of 15 bases or more, and in view of the exon skipping efficiency, the length is preferably 20 bases or more, more preferably 23 bases or more, and further more preferably 25 bases or more; and it has a length of 116 bases which is the same as that of the base sequence shown in SEQ ID NO: 1 or less, preferably 80 bases or less, more preferably 60 bases or less, and further more preferably 40 bases or less. Specifically, in view of the balance between the exon skipping efficiency and the economy, the length thereof is preferably 20 bases or more and 40 bases or less, more preferably 23 bases or more and 38 bases or less, further more preferably 25 bases or more and 35 bases or less.

### (Base)

The base (also referred to as nucleobase) constituting the base sequence of the antisense oligomer comprises natural bases of adenine (A), guanine (G), cytosine (C), thymine (T), uracil (U), and hypoxanthine (I), and non-natural modified base thereof. The ratio and the number of the modified bases comprised in the antisense oligomer are not particularly limited.

Examples of the modified base can comprise modified adenine such as 1-methyladenine, 2-methyladenine, N6-methyladenine, and 2-methylthio-N6-isopentenyladenine; modified guanine such as 2,2-dimethylguanine, 2-methylguanine, and 7-methylguanine; modified cytosine such as 5-methylcytosine, 4-acetylcytosine, 3-methylcytosine, and 2-thiocytosine; modified uracil such as uracil-5-oxyacetic acid, pseudouracil, 3-methyluracil, dihydrouracil, 5-ethyluracil, 5-bromouracil, 6-methyluracil, 2-thiouracil, 4-thiouracil, 5-(carboxy hydroxymethyl)uracil, 5'-carboxymethylaminomethyl-2-thiouracil, 5-carboxymethylaminomethyluracil, 5-methoxyaminomethyl-2-thiouracil, 5-methylaminomethyluracil, 5-methylcarbonylmethyluracil, 5-methyloxyuracil, and 5-methyl-2-thiouracil; modified hypoxanthine such as 1-methylhypoxanthine; and others such as 6-azapyrimidine, purine, 2,6-diaminopurine, 2-aminopurine, indole, imidazole, and xanthine.

### (Complementary Base Sequence)

The term "complementary base sequence" means a base sequence that forms Watson-Crick base pairs selected from the group consisting of adenine-thymine, adenine-uracil and guanine-cytosine, and corresponding base pairs with a base sequence of interest. In the Watson-Crick base pairs, a hydrogen bond between base pairs is formed. Here, the term "corresponding base pair" means a base pair that is formed by using, for example, the above-described modified base instead of adenine, thymine, uracil, guanine, or cytosine, corresponding to adenine-thymine, adenine-uracil, or guanine-cytosine; a wobble base pair such as guanine-uracil, inosine-uracil, inosine-adenine, and inosine-cytosine; and the like.

### (Contiguous Base Sequence of 15 or More Bases in Base Sequence Shown in SEQ ID NO: 1)

In the base sequence (i), the target sequence of the antisense oligomer is a contiguous base sequence of 15 or more bases in the base sequence shown in SEQ ID NO: 1. The base sequence shown in SEQ ID NO: 1 consists of a sequences of 3' 20 bases of intron 26, an entire base sequence of exon 27, and a sequence of 5' 20 bases of intron 27 in the human WRN gene.

SEQ ID NO: 1: ttaattttattattttttagTTCGAAAACTGTATCTTCGGGCACCAAAGAGCATT
GTTATAATCAAGTACCAGTTGAATTAAGTACAGAGAAGAAGgtttgttttaaagaaattgt

The target sequence of the antisense oligomer preferably comprises at least either of a base sequence of the boundary region between intron 26 and exon 27, and a base sequence of the boundary region between exon 27 and intron 27.

More specifically, the target sequence of the antisense oligomer preferably comprises at least either of the base sequence shown in SEQ ID NO: 2 and the base sequence shown in SEQ ID NO: 3. SEQ ID NO: 2 is a base sequence of the boundary region between intron 26 and exon 27, which consists of 3' one base of intron 26 and 5' eight bases of exon 27. Further, SEQ ID NO: 3 is a base sequence of the boundary region between exon 27 and intron 27, which consists of 3' eight bases of exon 27 and 5' one base of intron 27.
**SEQ ID NO: 2:** gTTCGAAAA
**SEQ ID NO: 3:** AGAAGAAGg

The target sequence of the antisense oligomer more preferably comprises at least either of the base sequence shown in SEQ ID NO: 4 and the base sequence shown in SEQ ID NO: 5. SEQ ID NO: 4 is a base sequence of the boundary region between intron 26 and exon 27, which consists of 3' four bases of intron 26 and 5' 17 bases of exon 27. Further, SEQ ID NO: 5 is a base sequence of the boundary region between exon 27 and intron 27, which consists of 3' 17 bases of exon 27 and 5' four bases of intron 27.
**SEQ ID NO: 4:**ttagTTCGAAAACTGTATCTT
**SEQ ID NO: 5:** TAAGTACAGAGAAGAAGgttt

Another preferable embodiment of a contiguous base sequence of 15 or more bases in the base sequence shown in SEQ ID NO: 1 comprises the following base sequences (a) to (c).
(a) a contiguous base sequence of 15 or more bases in the base sequence shown in SEQ ID NO: 1, in which any of the 1st, 2nd, 3rd, 4th, 5th, 6th, 7th, 8th, 9th, 10th, 11th, 12th, 13th, 14th, 15th, 16th, 17th, 18th, 19th, or 20th base in the base sequence shown in SEQ ID NO: 1 serves as the 5' starting point; and any of the 21st, 22nd, 23rd, 24th, 25th, 26th, 27th, 28th, 29th, 30th, 31st, 32nd, 33rd, 34th, 35th, 36th, 37th, 38th, 39th, 40th, 41st, 42nd, 43rd, 44th, 45th, 46th, 47th, 48th, 49th, 50th, 51st, 52nd, 53rd, 54th, 55th, 56th, 57th, 58th, 59th, or 60th base in the base sequence shown in SEQ ID NO: 1 serves as the 3' ending point,
(b) a contiguous base sequence of 15 or more bases in the base sequence shown in SEQ ID NO: 1, in which any of the 57th, 58th, 59th, 60th, 61st, 62nd, 63rd, 64th, 65th, 66th, 67th, 68th, 69th, 70th, 71st, 72nd, 73rd, 74th, 75th, 76th, 77th, 78th, 79th, 80th, 81st, 82nd, 83rd, 84th, 85th, 86th, 87th, 88th, 89th, 90th, 91st, 92nd, 93rd, 94th, 95th, or 96th base in the base sequence shown in SEQ ID NO: 1 serves as the 5' starting point; and any of the 97th, 98th, 99th, 100th, 101st, 102nd, 103rd, 104th, 105th, 106th, 107th, 108th, 109th, 110th, 111th, 112th, 113th, 114th, 115th, or 116th base in the base sequence shown in SEQ ID NO: 1 serves as the 3' ending point, and
(c) a contiguous base sequence of 15 or more bases in the base sequence shown in SEQ ID NO: 1, in which any of the 1st, 2nd, 3rd, 4th, 5th, 6th, 7th, 8th, 9th, 10th, 11th, 12th, 13th, 14th, 15th, 16th, 17th, 18th, 19th, or 20th base in the base sequence shown in SEQ ID NO: 1 serves as the 5' starting point; and any of the 97th, 98th, 99th, 100th, 101st, 102nd, 103rd, 104th, 105th, 106th, 107th, 108th, 109th, 110th, 111th, 112th, 113th, 114th, 115th, or 116th base in the base sequence shown in SEQ ID NO: 1 serves as the 3' ending point.

The above base sequence (a) comprises the boundary region between intron 26 and exon 27. The above base sequence (b) comprises the boundary region between exon 27 and intron 27. The above base sequence (c) comprises the boundary region between intron 26 and exon 27, the entire region of exon 27, and the boundary region between exon 27 and intron 27.

The target sequence of the antisense oligomer is, in view of the economy, preferably the above base sequence (a) or the above base sequence (b).

Specific examples of the target sequence of the antisense oligomer comprises the following base sequences. Note that SEQ ID NOs: 6 to 10 are base sequences that target the boundary region between intron 26 and exon 27, and SEQ ID NOs: 11 to 15 are base sequences that target the boundary region between exon 27 and intron 27.
**SEQ ID NO: 6:** TTTTTTAGTTCGAAAACTGTATCTTCGGGC
**SEQ ID NO: 7:** TTTTTAGTTCGAAAACTGTATCTTCGGGCA
**SEQ ID NO: 8:** TTTTAGTTCGAAAACTGTATCTTCGGGCAC
**SEQ ID NO: 9:** TTTAGTTCGAAAACTGTATCTTCGGGCACC
**SEQ ID NO: 10:** TTAGTTCGAAAACTGTATCTTCGGGCACCA
**SEQ ID NO: 11:** TGAATTAAGTACAGAGAAGAAGGTTTGTTT
**SEQ ID NO: 12:** TTGAATTAAGTACAGAGAAGAAGGTTTGTT
**SEQ ID NO: 13:** GTTGAATTAAGTACAGAGAAGAAGGTTTGT
**SEQ ID NO: 14:** AGTTGAATTAAGTACAGAGAAGAAGGTTTG
**SEQ ID NO: 15:** CAGTTGAATTAAGTACAGAGAAGAAGGTTT
**SEQ ID NO: 41:** TGAATTAAGTACAGAGAAGAAGGTTTGT

### (Deletion, Substitution or Insertion)

The above base sequence (ii) has deletion, substitution, or insertion of one or more bases in a contiguous base sequence of 15 or more bases in the base sequence shown in SEQ ID NO: 1.

The number of bases deleted, substituted or inserted is not limited as long as the effect of the present invention is not impaired. For example, the number is 1 or more, and preferably 5 or less, more preferably 3 or less, and further more preferably 2 or less. That is, the number of bases deleted, substituted or inserted is preferably 1, 2, 3, 4 or 5, more preferably 1, 2, or 3, further more preferably 1 or 2, and yet further more preferably 1.

In a base sequence, the base sequence having deletion, substitution or insertion of one or more bases comprises a sequence having deletion, substitution or insertion of a given number (e.g., 1 or more and 5 or less) of bases in the base sequence, and being capable of sufficiently specifically hybridizing with high efficiency under stringent conditions to a complementary sequence of the base sequence. However, it is not limited to this. The term the "stringent condition" refers to a condition where only specific hybridization occurs and non-specific hybridization does not occur; and conditions normally used in the art can be optionally selected depending on the base length of interest. Among deletion, substitution or insertion, substitution is preferable in view of the exon skipping efficiency.

Patients with Werner syndrome to be treated may have base polymorphism with respect to a reference base sequence. A preferred example of the deletion, substitution or insertion in the above (ii) is deletion, substitution or insertion to match the polymorphism which a Werner syndrome patient has, to the sequence of the antisense oligomer. The base polymorphism which a Werner syndrome patient to be treated has can be obtained by analyzing the genomic sequence of the patient. The base polymorphism in exon 27 can be confirmed by reference to publicly available database (e.g.,
URL:https://ensembl.org) .

### <Antisense Oligomer>

The antisense oligomer of the present invention is not limited as long as it has the above specified base sequence and capable of causing skipping of exon 27 in a human WRN gene. For example, the antisense oligomer is any of an oligonucleotide, a morpholino oligomer, a peptide nucleic acid (PNA) oligomer, or a glycol nucleic acid (GNA) oligomer.

### (Oligonucleotide)

The oligonucleotide is an oligomer to which a nucleotide having a base moiety, a sugar moiety and a phosphate moiety as its basic structure is linked by a phosphate bond. The nucleotide may be any of a natural nucleotide and a non-natural nucleotide.

Examples of the natural nucleotide comprise a deoxyribonucleotide in which the sugar moiety is deoxyribose and a ribonucleotide in which the sugar moiety is ribose; and the respective oligomers are natural DNA and RNA. The phosphate bond between the nucleotides in DNA and RNA is a phosphodiester bond.

One or more of the base moiety, the sugar moiety and the phosphate moiety in the non-natural nucleotide has a non-natural modification. Accordingly, the antisense oligomer of the present invention may comprise at least one base modification, and/or at least one sugar modification, and/or at least one phosphate moiety (structure) modification.

Non-natural modified bases are described above.

Examples of the non-natural sugar moiety comprise a bridged nucleic acid (BNA) (e.g., a linked nucleic acid (LNA), an amide-bridged nucleic acid (AmNA), a guanidine-bridged nucleic acid (GuNA), a spirocyclopropylene bridged nucleic acid (scpBNA)and the like), a cyclohexene nucleic acid (CeNA), a 1,5-anhydrohexitol nucleic acid (HNA), a 2'-0,4'-C-ethylene-bridged nucleic acid (ENA), a constrained ethyl-bridged nucleic acid (cEtBNA), a threose nucleic acid (TNA), a fluoro-β-arabino nucleic acid (FNA), tricyclo DNA (tcDNA), a 2'-F modified ribonucleic acid (2'-F-RNA), a 2'-O-methyl modified ribonucleic acid (2'-O-Me-RNA), a 2'-O-methoxyethyl modified ribonucleic acid (2'-O-MEO-RNA), a 4'-thio modified ribonucleic acid (4'-S-RNA or 4'-S-DNA) and the like.

Examples of the oligonucleotide having a non-natural phosphate bond comprise those in which a natural phosphodiester bond is partially or fully substituted with a non-natural phosphate bond selected from a phosphorothioate bond (sulfurization), a phosphorodithioate bond, an alkylphosphonate bond, a phosphoramidate bond, a phosphorodiamidate bond and a boranophosphate bond.

### (Morpholino Oligomer)

The morpholino oligomer is preferably an oligomer (phosphorodiamidate morpholino oligomer (PMO)) to which the group represented by the following formula (I) is linked. 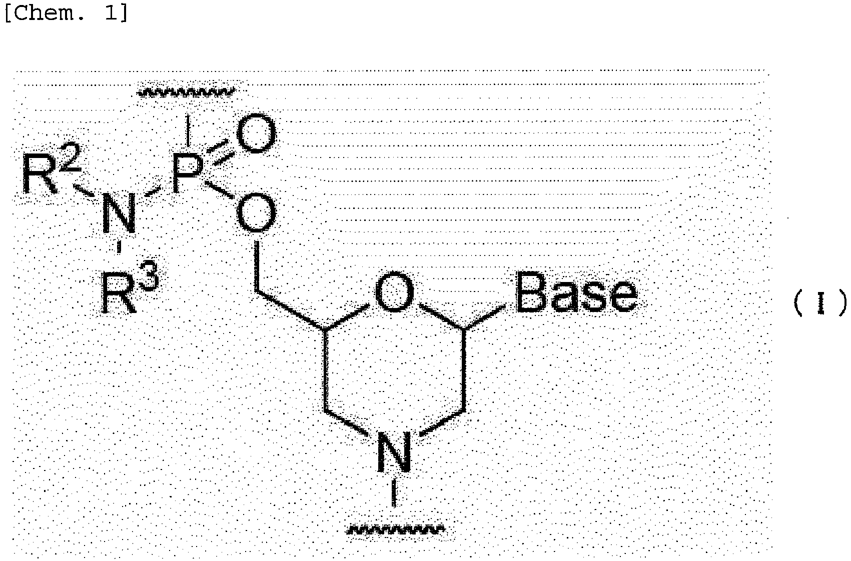 wherein R² and R³ are the same or different, and represent a hydrogen atom, linear or branched alkyl having 1 or more and 6 or less carbon atoms, cycloalkyl having 5 or more and 12 or less carbon atoms, or aryl having 6 or more and 10 or less carbon atoms.

Alkyl is preferably linear or branched alkyl having 1 or more and 6 or less carbon atoms. Specific examples thereof comprise methyl, ethyl, n-propyl, isopropyl, n-butyl, isobutyl, sec-butyl, tert-butyl, n-pentyl, isopentyl, neopentyl, tert-pentyl, n-hexyl, and isohexyl. Alkyl may have 1 or more and 3 or less substituents.

Cycloalkyl is preferably cycloalkyl having 5 or more and 12 or less carbon atoms. Specific examples thereof comprise cyclopentyl, cyclohexyl, cycloheptyl, cyclooctyl, cyclodecyl, and cyclododecyl, for example.

Aryl is preferably aryl having 6 or more and 10 or less carbon atoms. Specific examples thereof can comprise phenyl, α-naphthyl, and β-naphthyl, for example. Especially, phenyl is preferable among others. Aryl may have 1 or more and 3 or less substituents.

Examples of the above substituents comprise a halogen atom, alkoxy, a cyano group, and a nitro group and the like.

Examples of the halogen atom comprise a fluorine atom, a chlorine atom, a bromine atom, an iodine atom.

Examples of alkoxy can comprise linear or branched alkoxy having 1 or more and 6 or less carbon atoms, for example, methoxy, ethoxy, n-propoxy, isopropoxy, n-butoxy, isobutoxy, sec-butoxy, tert-butoxy, n-pentyloxy, isopentyloxy, n-hexyloxy, and isohexyloxy, and the like. Alkoxy having 1 or more and 3 or less carbon atoms is preferable among others.

The morpholino oligomer can be produced according to WO 1991/009033 or WO 2009/064471, for example. Particularly, PMO can be produced according to the methods described in WO 2009/064471 or WO 2013/100190.

### (Modification of 5'-End and 3'-End)

At least one of the 5'-end and the 3'-end of the oligomer of the present invention may be modified. In one embodiment, both the 5'-end and the 3'-end may be modified.

Examples of modifying groups of the 5'-end and/or the 3'-end comprise an α-tocopherol group, a polyethylene glycol (PEG) group, an N-acetylglucosamine (O-GlcNAc) group, a methoxy (O-Me) group, a cholesterol group, an amino group, a peptide group (e.g., cell-penetrating peptide described later) and derivatives thereof, and the like. The modifying group may be bound to the 5'-end or the 3'-end of the oligomer directly or via a linking part.

Examples of the linking part is not particularly limited as long as it can covalently link a desired modifying group with the 5'-end or the 3'-end of the oligonucleotide, and the linking groups used in the art, such as -O-, -CO-, -NH-, -CONH-, and -NHCO- can be used.

One embodiment of the 5'-end of the oligonucleotide of the present invention can include the following group (1) or (2): 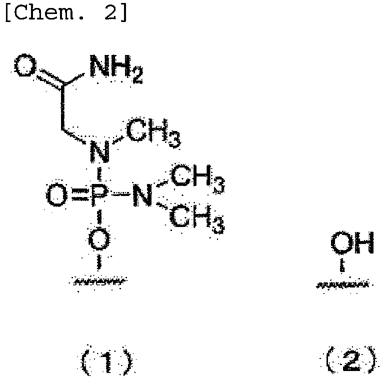 wherein the wavy line indicates the bond to the 5'-end of the oligonucleotide.

In another embodiment, the 5'-end is modified with the group having the following structure (6): 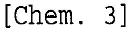 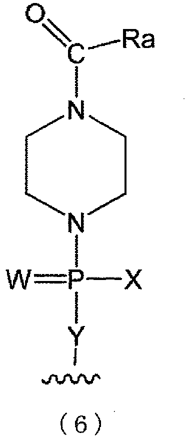 wherein
the wavy line indicates the bond to the 5'-end of the oligonucleotide,
W is S or O, and preferably O,
X is NR^{A1}R^{A2} (R^{A1} and R^{A2} are each independently a hydrogen atom or lower alkyl, for example, methyl.) or OR^{A3} (R^{A3} is a hydrogen atom or lower alkyl, for example, methyl.),
Y is O or NR^{A4} (R^{A4} is a hydrogen atom or lower alkyl, for example, methyl.), and
Ra is any modifying group (e.g., a PEG group, a cholesterol group, an amino group, a peptide group or a derivative thereof).

The group having the above structure (6) is preferably a group having the following structure (6-1).

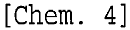

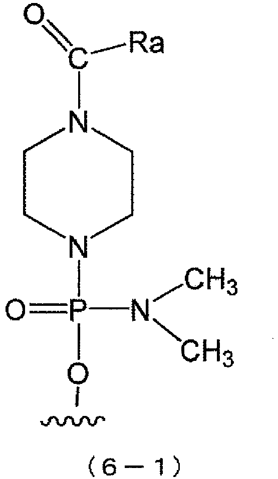

Ra is a group represented by the following (3), (4), (5), or (7) .

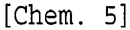

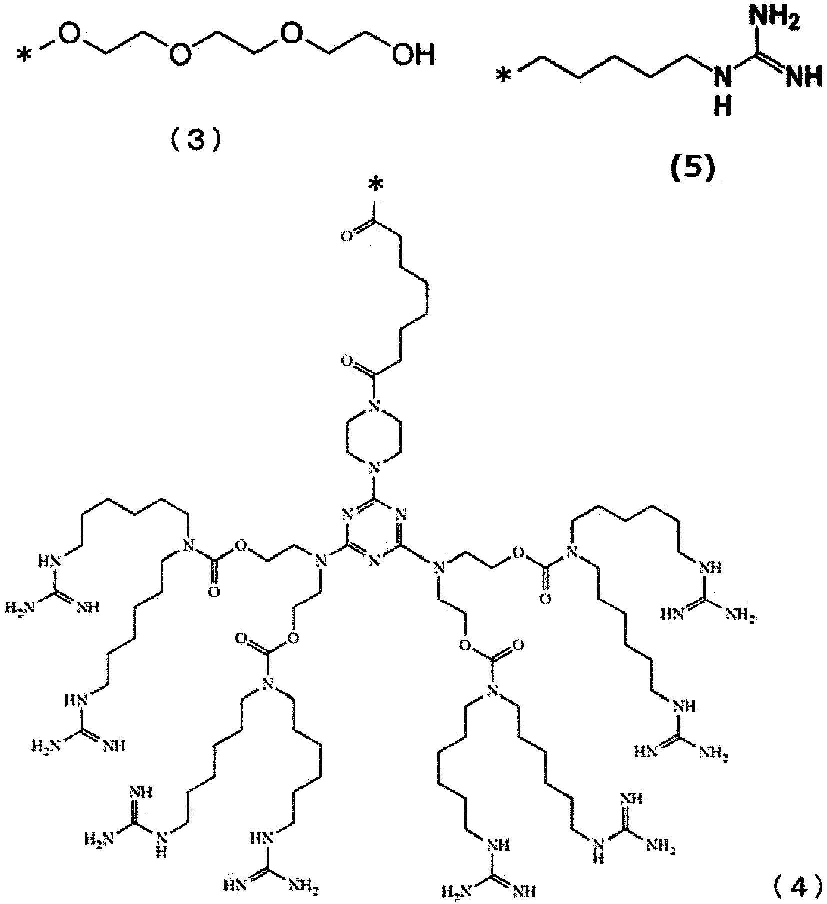

*-L1-peptide group (7)
wherein * indicates the bond to the above structure (6) or (6'), and L1 represents a single bond or a linker.

The linker represented by L1 is not particularly limited as long as the group having the structure (6) or (6') can be bound to the peptide via the linker, and examples thereof can comprise a group represented by any of the following (8-1) to (8-3), for example.

### [Chem. 6]

*-(CH₂)ₙ-** (8-1)

*-(CH₂)ₙ-NHCO-(CH₂)ₘ-** (8-2)

*-(CH₂)ₙ-CONH-(CH₂)ₘ-** (8-3)

wherein * is the same as described above, ** indicates the bond to the peptide, n represents an integer from 2 to 6, and m represents an integer from 2 to 6.

Examples of the peptide moiety of the above peptide group comprise cell-penetrating peptides (CPPs). Specific examples of CPP comprise cationic CPPs such as TAT, R8, DPV3, DPV6, penetratin, and R9-TAT; amphipathic CPPs such as pVEC, ARF (19-31), MPG, MAP, and transportan; and hydrophobic CPPs such as Bip4, C105Y, melittin, and gH625. CPP can be appropriately designed based on the literatures by those skilled in the art (e.g., Xie J, et al., Front Pharmacol. 2020 May 20; 11:697).

One embodiment of the 3'-end can comprise the following group (2'), (4'), or following (9), for example.

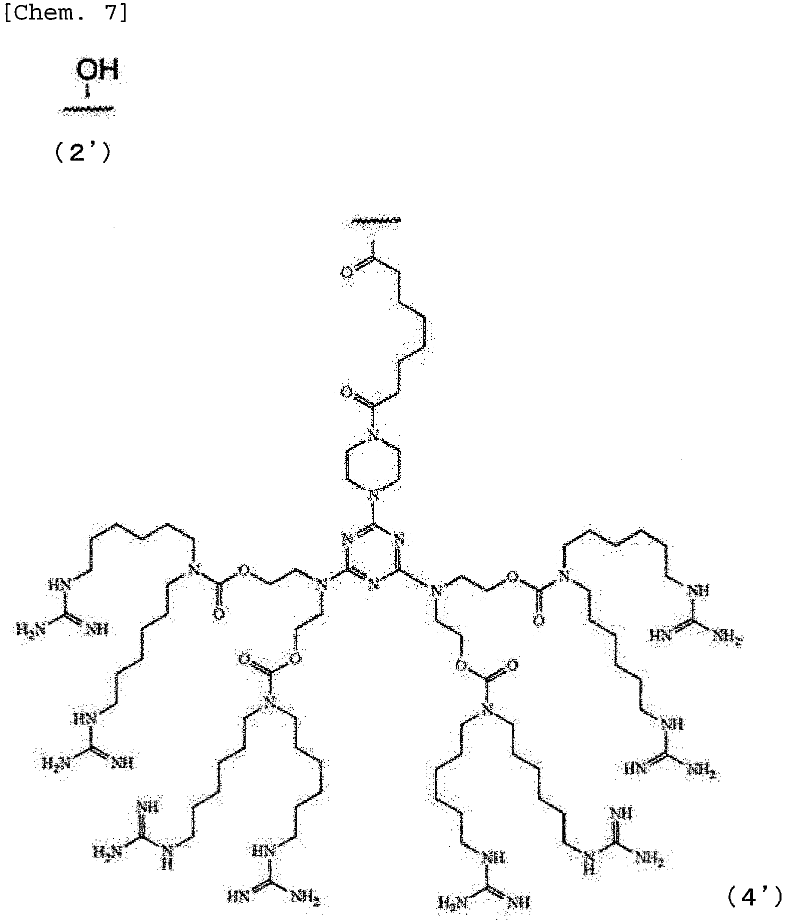

#-L2-peptide group (9)
wherein # indicates the bond to the 3'-end of the oligonucleotide, and L2 represents a single bond or a linker.

The linker represented by L2 is not particularly limited as long as the sugar of the 3'-end of the oligonucleotide can be bound to the peptide via the linker, and examples thereof can comprise a group represented by the following groups (10-1) to (10-3).

### [Chem. 8]

Examples include groups represented by:

^{#}-(CH₂)ₙ-^{##} (10-1)

^{#}-(CH₂)ₙ-NHCO-(CH₂)ₘ-^{##} (10-2)

^{#}-(CH₂)ₙ-CONH-(CH₂)ₘ-^{##} (10-3)

wherein # is the same as described above, ## indicates the bond to the peptide group, n represents an integer from 2 to 6, and m represents an integer from 2 to 6.

For the peptide moiety of the above peptide group, the same one as for the modification to the 5'-end can be used.

As one embodiment of the oligomer of the present invention, Vivo-Morpholinos (manufactured by Gene Tools, LLC), which is a modified morpholino oligomer, can be used. Vivo-Morpholinos have the above modifying group (1) at the 5'-end, and the above modifying group (4) (octa-guanidine dendrimer) at the 3'-end.

### (Method of Producing Various Antisense Oligomers)

The various antisense oligomers described above can be produced by a known technique or a novel technique which applies the known technique based on the base sequence information. The antisense oligomer can be produced by entrusting to a third-party organization.

### <Pharmaceutically Acceptable Salt>

In the present invention, the antisense oligomer may be a pharmaceutically acceptable salt.

Examples of the salt comprise an acid addition salt and a base addition salt.

The acid addition salt may be either of an inorganic acid salt and an organic acid salt. Examples of the inorganic acid salt comprise hydrochloride, hydrobromide, sulfate, hydroiodide, nitrate, and phosphate, for example. Examples of the organic acid salt comprise citrate, oxalate, acetate, formate, propionate, benzoate, trifluoroacetate, maleate, tartrate, methanesulfonate, benzenesulfonate and para-toluenesulfonate, for example.

The base addition salt may be either of an inorganic base salt and an organic base salt. Examples of the inorganic base salt comprise sodium salt, potassium salt, calcium salt, magnesium salt, and ammonium salt, for example. Examples of the organic base salt comprise triethylammonium salt, triethanolammonium salt, pyridinium salt, and diisopropylammonium salt, for example.

The compound of the present invention may be a solvate such as a hydrate. The solvent is not particularly limited as long as it is a pharmaceutically acceptable solvent.

### [Viral Vector]

The viral vector of the present invention is a viral vector which expresses RNA capable of causing skipping of the 27th exon in a human WRN gene, said RNA consists of a base sequence complementary to the base sequence of the following (i) or (ii):
(i) a contiguous base sequence of 15 or more bases in the base sequence shown in SEQ ID NO: 1; or
(ii) a contiguous base sequence of 15 or more bases in the base sequence shown in SEQ ID NO: 1, having deletion, substitution, or insertion of one or more bases.

The base sequence is the same as those of the above antisense oligomer.

Provided that the base length of RNA which the viral vector expresses is 15 bases or more, and in view of the exon skipping efficiency, preferably 20 bases or more, more preferably 40 bases or more, and further more preferably 60 bases or more; and the number of bases is the same as that of the base sequence shown in SEQ ID NO: 1 or less.

The target sequence of the viral vector preferably comprises the base sequence of the boundary region between intron 26 and exon 27, the full length of exon 27, and the base sequence of the boundary region between exon 27 and intron 27.

More specifically, the target sequence of the viral vector preferably comprises the base sequence shown in SEQ ID NO: 16. SEQ ID NO: 16 is a base sequence consisting of 3' one base of intron 26, the full length of exon 27, and 5' eight bases of exon 27.

SEQ ID NO: 16: gTTCGAAAACTGTATCTTCGGGCACCAAAGAGCATTGTTATAATCAAGTACCAGT
TGAATTAAGTACAGAGAAGAAGg

The target sequence of the viral vector is, in view of the exon skipping efficiency, preferably the above base sequence (c) comprising the boundary region between intron 26 and exon 27, the entire region of exon 27, and the boundary region between exon 27 and intron 27.

The viral vector can express one or two or more of RNA. When it expresses one RNA, the RNA preferably comprises the base sequence of the boundary region between intron 26 and exon 27, the entire region of exon 27, and the base sequence of the boundary region between exon 27 and intron 27. When it expresses two or more RNAs, it is preferable to express one or more RNAs having the base sequence of the boundary region between intron 26 and exon 27, and one or more RNAs having the base sequence of the boundary region between exon 27 and intron 27. When it expresses two or more RNAs, it is preferable to use two or more viral vectors in combination, each of which has a cassette for separately expressing antisense oligomers.

The type of the viral vector is not limited. Examples thereof comprise a retrovirus, a lentivirus, an adenovirus, and an adeno-associated virus, for example. Examples of the serotype of the adeno-associated virus comprise AAV1, AAV2, AAV3, AAV4, AAV5, AAV6, AAV7, AAV8, AAV9, AAV10, AAV-PHP.B, AAV-PHP.eB, AAV-DJ, and AAV-DJ/8, and the like.

The production of a viral vector having a desired sequence thereon can be performed by referring to a known method in the art as appropriate.

### [Pharmaceutical Composition]

The present invention provides a pharmaceutical composition comprising the above antisense oligomer or the above viral vector as an active ingredient.

In one preferable embodiment, the pharmaceutical composition is a pharmaceutical composition for the treatment of Werner syndrome. Another preferable embodiment of the pharmaceutical composition is a pharmaceutical composition for inducing skipping of exon 27 in a human WRN gene.

### <Active Ingredient>

The pharmaceutical composition comprises as an active ingredient the above antisense oligomer or the above viral vector.

It can comprise as an active ingredient one or two or more of the above antisense oligomers or the above viral vectors.

### <Carrier>

The pharmaceutical composition is provided as a pharmaceutical composition comprising one or two or more of pharmaceutically acceptable carriers, as necessary.

Examples of the carrier comprise lipid nanoparticles (LNP). Examples of the lipid constituting the lipid nanoparticles comprise a cationic lipid, a positively or negatively charged phospholipid, sterol, a saturated or unsaturated fatty acid, and a combination thereof. When the carrier is lipid nanoparticles, the antisense oligomer or the viral vector which serves as an active ingredient is preferably contained in the lipid nanoparticles.

Examples of the carrier can further comprise an aqueous buffer solution, a pH adjuster such as an acid or base, a stabilizer such as an ascorbic acid or p-amino benzoic acid, an excipient such as D-mannitol, an isotonizing agent, and a preservative, and the like.

### <Dosage Form>

The dosage form is not particularly limited, and examples thereof can comprise various injections, oral preparations, infusions, inhalants, ointments, lotions, sprays, and the like.

In addition, the pharmaceutical composition can be provided in any form of aqueous solution, frozen solution and lyophilizate.

### <Content of Active Ingredient>

The content of the active ingredient in the pharmaceutical composition is not particularly limited. The content of the active ingredient can be appropriately set according to the type of the active ingredient, use, dosage form, and the type of the carrier. The content of the active ingredient in the pharmaceutical composition is, for example, 0.0001% by mass or more, preferably 0.001% by mass or more, and more preferably 0.01% by mass or more; and 100% by mass or less, preferably 90% by mass or less, and more preferably 50% by mass or less.

### <Subject to Be Administered>

The pharmaceutical composition is to be administered to Werner syndrome patients and carriers. Specifically, subjects to be administered are Werner syndrome patients and carriers who carry homozygous or heterozygous c.3139-1G>C mutation in a human WRN gene.

The race, gender, and age of the subjects to be administered are not particularly limited.

Note that approximately 60% of the patients with Werner syndrome reported globally are Japanese. Further, 70.7% of the patients with Werner syndrome reported in Japan have c.3139-1G>C mutation in the human WRN gene.

The patients with Werner syndrome exhibit symptoms such as progeroid hair changes (e.g., gray hair, baldness), ocular cataracts (bilateral), skin atrophy/skin sclerosis (e.g., corns, callus), refractory ulceration, soft-tissue calcifications (e.g., Achilles tendons), disorder of glucose/lipid metabolism, premature arteriosclerosis (e.g., angina pectoris, myocardial infarctions). The subjects to be administered may be patients who exhibit these symptoms or patients and carriers who do not exhibit symptoms yet.

### <Administration Route>

Examples of the administration route of the pharmaceutical composition comprise parenteral administration such as transdermal, subcutaneous, intramuscular, intravenous, and intraarterial administration, and oral administration. The administration route may depend on symptoms and, for example, may be an administration route suitable for the sites exhibiting aging signs.

For example, patients with symptoms of ulceration can be administered transdermally such as by ointments, lotions, or sprays.

### <Amount to Be Administered>

The amount to be administered can be appropriately set by those skilled in the art.

When the active ingredient is the antisense oligomer, the amount of the antisense oligomer to be administered is preferably 0.1 mg/kg or more, more preferably 1 mg/kg or more, and further more preferably 10 mg/kg or more, and preferably 1000 mg/kg or less, more preferably 500 mg/kg or less, and further more preferably 100 mg/kg or less. When administering locally, the amount is preferably 0.01 µg or more, and more preferably 1 µg or more; and preferably 1000 µg or less, and more preferably 100 µg or less. When administering systemically, the amount is preferably 0.1 mg/kg or more, and more preferably 1 mg/kg or more; and preferably 500 mg/kg or less, and more preferably 100 mg/kg or less.

When the active ingredient is the antisense oligomer, the administration can be performed from once to several times daily, or at intervals of one day or several days.

When the active ingredient is the viral vector, the amount of the viral vector to be administered is preferably 1 × 10¹² vg/kg or more, more preferably 1 × 10¹³ vg/kg or more, and further more preferably 2 × 10¹³ vg/kg or more; and preferably 1 × 10¹⁶ vg/kg or less, more preferably 1 × 10¹⁵ vg/kg or less, and further more preferably 5 × 10¹⁴ vg/kg or less.

When the active ingredient is the viral vector, the administration can be performed once or several times.

### [Method for Treatment]

The present invention also provides a method for treating Werner syndrome, comprising a step of administering the effective amount of the above antisense oligomer or the above viral vector to a Werner syndrome patient and carrier.

Specific active ingredient, subject to be administered, amount to be administered and the like are as described above.

### Examples

Hereinafter, the present invention will be specifically described by referring to Examples.

The cell culture in Examples was conducted under wet environment of 37°C and 5% carbon dioxide.

### Production Example 1: Production of Morpholino Antisense Oligomer (1)

The morpholino antisense oligomers with the sequences shown in Table 1 below were synthesized. The synthesis was entrusted to a synthesis service provided by Gene Tools, LLC.

**[Table 1]**

| Table 1: Antisense oligomer sequences (1) | |
|---|---|
| AS01 : | CGAAGATACAGTTTTCGAACTAAAA [hWRN I26E27 25nt M0] (SEQ ID NO: 17) |
| AS02 : | ACAAACCTTCTTCTCTGTACTTAAT [hWRN E27I27 25nt M0] (SEQ ID NO: 18) |
| AS03 : | ACAAACCTTCTTCTCTGTACTTAATTCAAC [hWRN E27I27 30 M0] (SEQ ID NO: 19) |
| AS04 : | CCTCTTACCTCAGTTACAATTTATA [Control M0] (SEQ ID NO: 20) |

### Production Example 2: Production and Culture Method of WS-iPS Cell Lines

Peripheral blood mononuclear cells were collected from a Werner patient carrying homozygous c.3139-1G>C mutation in intron 25 of the human WRN gene, and iPS cell lines were established in accordance with the description in Non-Patent Literature 2 to obtain WS-iPS cell lines. To culture iPS cell lines, a growth medium for iPS/ES cells "StemFit AK02N" (manufactured by Ajinomoto Healthy Supply Co., Inc.), a substrate for culture "iMatrix-511" (manufactured by Nippi, Inc.), and ROCK inhibitor Y-27632 (manufactured by FUJIFILM Wako Pure Chemical Corporation) were used.

### Production Example 3: Production of WS-WRN gene corrected iPS Cell Lines

In the WS-iPS cell lines obtained in Production Example 2, a CRISPR/Cas9 homology directed repair (HDR) genome editing technique was used, and cell lines in which c.3139-1G>C mutation in intron 25 of the human WRN gene was repaired to wild-type were established to obtain WS-WRN gene corrected iPS cell lines in accordance with the description of Non-Patent Literature 2.

### Production Example 4: Production of WS-iMSC Cell Lines

The WS-iPS cell lines obtained in Production Example 2 were allowed to form embryonic bodies, and then cultured for 5 days on a 60 mm culture dish (manufactured by Corning Incorporated) coated with type IV collagen by using a mesenchymal stem cell differentiation medium. After that, the culture was kept on a 100 mm culture dish coated with collagen I (manufactured by Nitta Gelatin Inc.) by using a culture medium to obtain WS-iMSC cell lines, which are mesenchymal stem cells derived from iPS cells.

The compositions of the mesenchymal stem cell differentiation medium and culture medium used were as follows.

### [Mesenchymal Stem Cell Differentiation Medium]

α-MEM (manufactured by Thermo Fisher, Inc.)
10% fetal bovine serum (FBS; manufactured by Thermo Fisher, Inc.)
100 nM dexamethasone (manufactured by Merck KGaA)
50 µM L-ascorbic acid 2-phosphate sesquimagnesium salt hydrate (manufactured by Merck KGaA)

### [Culture Medium]

### α-MEM

10% fetal bovine serum (FBS; manufactured by Thermo Fisher, Inc.)
0.1 mM non-essential amino acid (manufactured by Thermo Fisher, Inc.)

### Production Example 5: Production of Antisense Oligo-Expressing Lentivirus Vector Plasmid

Oligo DNAs with the sequences shown in Table 2 below were synthesized, a mixed solution of equivalent amount of the resulting oligo DNAs were heated at 99°C for 15 minutes followed by cooling to room temperature for 2 hours, and then the oligo DNAs were annealed to obtain a double-stranded DNA. The double-stranded DNA thus obtained was subjected to phosphorylation at its end by using T4 Polynucleotide Kinase (manufactured by New England Biolabs Inc.), and ligated into a lentivirus vector pLKO.1 puro digested with restriction enzymes AgeI and EcoRI. The lentivirus vector pLKO.1 puro was obtained from Addgene, a nonprofit plasmid repository. Its transformation was performed into E. Coli strain DH5a and the plasmid was purified from the obtained E. Coli strain with NucleoBond Midi (manufactured by Takara Bio Inc.) to obtain an antisense oligo-expressing lentivirus vector plasmid (pLenti-anti-WRN-Ex27).

**[Table 2]**

| Table 2: Oligo DNA sequences for lentivirus |
|---|
| Oligo DNA 1: |
| CCGGTAAAACAAACCTTCTTCTCTGTACTTAATTCAACTGGTACTTGATTATAACAATGCTCTTTGGTGCCCGAAGATACAGTCT TCGAACTAAAAAATATTTTT (SEQ ID NO: 21) |
| Oligo DNA 2: |
| AATTAAAAATATTTTTTAGTTCGAAGACTGTATCTTCGGGCACCAAAGAGCATTGTTATAATCAAGTACCAGTTGAATTAAGTAC AGAGAAGAAGGTTTGTTTTA (SEQ ID NO: 22) |

### Production Example 6: Production of Antisense Oligo-Expressing Lentivirus Vector

HEK 293 cells were cultured in DMEM medium with 10% FBS, and transfected with 3 plasmids: pCMV-VSV-G, psPAX2, and pLenti-anti-WRN-Ex27, which was obtained in Production Example 5, using Lipofectamine 2000 reagent (manufactured by Thermo Fisher Scientific, Inc.). The medium was exchanged 24 hours after the transfection, and the culture supernatant was recovered 48 hours after the transfection. The resulting virus solution was transferred to Amicon Ultra centrifugal Filters (manufactured by MilliporeSigma) and centrifuged at 5000 rpm for 30 minutes to obtain a concentrated virus solution. HEK 293 was infected with the antisense oligo-expressing lentivirus vector using the obtained concentrated virus solution. Then, confirmation of the infection ability and the calculation of titers of the concentrated virus solution were performed and hence, the produced viral vector was confirmed to have sufficient infection ability.

### Production Example 7: Production of Morpholino Antisense Oligomer (2)

Morpholino antisense oligomers with the sequences shown in Table 3 below were synthesized. The synthesis was entrusted to a synthesis service provided by Gene Tools, LLC.

ASO 15 and ASO 16 are Vivo-Morpholinos.

**[Table 3]**

| Table 3: Antisense oligomer sequences (2) | |
|---|---|
| AS05 : | GCCCGAAGATACAGTTTTCGAACTAAAAAA [WRN_I26E27#1_30nt] (SEQ ID NO: 25) |
| AS06 : | TGCCCGAAGATACAGTTTTCGAACTAAAAA [WRN_I26E27#2_30nt] (SEQ ID NO: 26) |
| AS07 : | GTGCCCGAAGATACAGTTTTCGAACTAAAA [WRN_I26E27#3_30nt] (SEQ ID NO: 27) |
| AS08 : | GGTGCCCGAAGATACAGTTTTCGAACTAAA [WRN_I26E27#4_30nt] (SEQ ID NO: 28) |
| AS09 : | TGGTGCCCGAAGATACAGTTTTCGAACTAA [WRN_I26E27#5_30nt] (SEQ ID NO: 29) |
| AS010 : | AAACCTTCTTCTCTGTACTTAATTCAACTG [WRN_E27I27#1_30nt](SEQ ID NO: 30) |
| AS011 : | CAAACCTTCTTCTCTGTACTTAATTCAACT [WRN_E27I27#2_30nt] (SEQ ID NO: 31) |
| AS012 : | ACAAACCTTCTTCTCTGTACTTAATTCA [WRN_E27127#3_28nt] (SEQ ID NO: 32) |
| AS013 : | AAGAAACCTTCTTCTCTGTACTTAATTCAA [WRN E27I27#4_30nt] (SEQ ID NO: 33) |
| AS014 : | AAACAAACCTTCTTCTCTGTACTTAATTCA [WRN_E27127#5_30nt] (SEQ ID NO: 34) |
| AS015 : | CCTCTTACCTCAGTTACAATTTATA [Vivo Standard Control] (SEQ ID NO: 35) |
| AS016 : | ACAAACCTTCTTCTCTGTACTTAATTCA [Vivo WRN_E27I27#3_28nt] (SEQ ID NO: 36) |

### Example 1: Exon Skipping in iPS Cells (1) (Morpholino Antisense Oligomer)

The WS-iPS cells obtained in Production Example 2 and the WS-gene corrected iPS cells obtained in Production Example 3 were seeded in a 24-well plate, and after 24 hours, ASO1, ASO2, ASO3, and ASO4 obtained in Production Example 1 were each added at a concentration of 10 µM to the cells using Endo-Porter reagent (manufactured by Gene Tools, LLC).

Thereafter, the culture was performed for 24 hours.

### Test Example 1-1: RT-PCR Analysis

The cells were harvested and total RNA was purified using "PureLink RNA Mini Kit" (manufactured by Thermo Fisher, Inc.). Total RNA was used as a template and reverse transcription was performed using Superscript III reagent (manufactured by Thermo Fisher, Inc.) to synthesize cDNA. Further, the obtained cDNA was used as a template, and polymerase chain reaction (PCR) was performed using the primer pair shown in Table 4 below. Primer 1 is a primer for exon 25, and Primer 2 is a primer for exon 28.

**[Table 4]**

| Table 4: Primer sequences for RT-PCR | |
|---|---|
| Primer 1: | TGGCACTGGCAAGGATCAAA (SEQ ID NO: 23) |
| Primer 2: | TCCCAGAAGAAATCTTATCACATGG (SEQ ID NO: 24) |

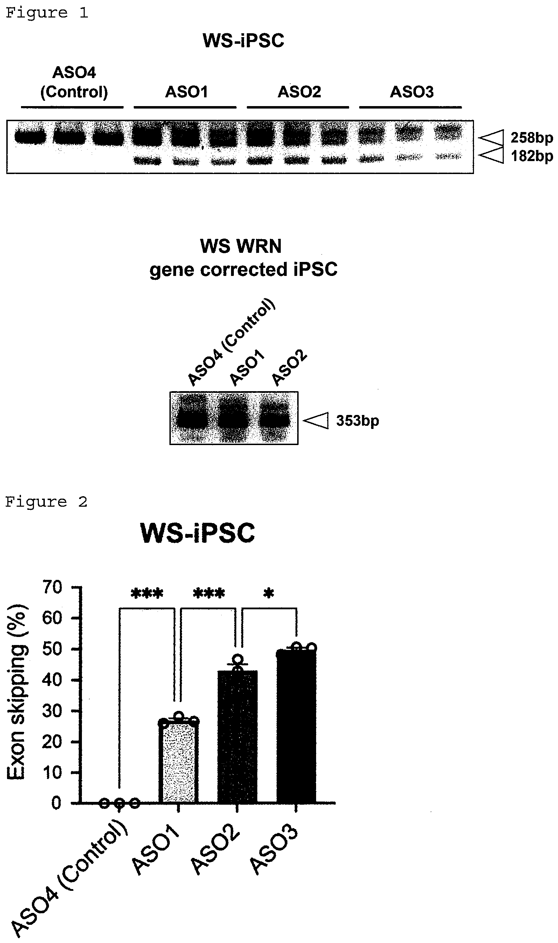

The obtained PCR amplified product was analyzed by 1% agarose gel electrophoresis. The results are shown in Figure 1.

As shown in Figure 1, a 258 bp mutant band with lack of exon 26 was observed in the WS-iPS cells to which the control, ASO4, was added.

On the other hand, a 182 bp mutant band which was truncated due to lack of exon 27 as well as exon 26 in the human WRN gene by exon skipping was observed in the WS-iPS cells to which ASO1, ASO2, or ASO3 was added.

The above results indicate that the addition of each of ASO1 and ASO2 can induce exon skipping of exon 27 in a human WRN gene.

Note that no difference in band was observed between the case of adding ASO1 or ASO2 and in the case of adding the control ASO4 in the WS-WRN gene corrected iPS cells.

The results of 1% agarose gel electrophoresis of the obtained PCR amplified product were analyzed with Image J image analysis software (manufactured by the US National Institute of Health (NIH)), and the exon skipping efficiency was quantified. The results are shown in Figure 2.

The exon skipping efficiencies of ASO1, ASO2, and ASO3 which were quantified based on band brightness were 27.9 ± 0.7%, 43.0 ± 2.1%, and 49.7 ± 0.8%, respectively. The above results indicate that the addition of each of ASO1, ASO2, and ASO3 can induce exon skipping of exon 27 in the splicing of the WRN gene in human WS patients, and as a result, the stop codon generated in exon 27 no longer functions, thereby leading to gene expression of mRNA up to the stop codon of normal WRN gene. In addition, in the case of skipping of exon 27 using the antisense oligomer, the expression level of truncated mRNA up to the stop codon of the WRN gene was confirmed to be expressed at almost the same level as the one in the case of hetero mutation, which does not develop Werner syndrome (half the expression level compared to the normal case).

### Test Example 1-2: Western Blotting Analysis

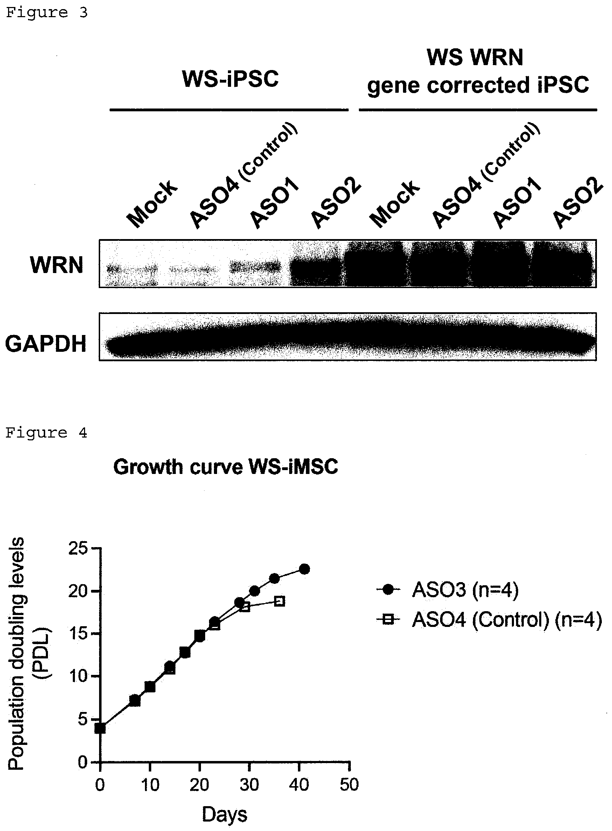

The cells were harvested, and the expression of human WRN proteins was confirmed by Western blotting method. The results are shown in Figure 3. For detection, anti-WRN antibody (manufactured by Abeam plc, ab66606), an anti-GAPDH antibody (manufactured by Cell Signaling Technology, Inc., #5174), second antibodies (manufactured by GE healthcare technologies Inc., #NA931, #NA934) were each used.

As shown in Figure 3, in the WS-iPS cells with ASO2 added, the prominent expression of a protein which molecular weight was smaller than that of the wild-type WRN protein was observed. Further, in the WS-iPS cells with ASO1 added, the insignificant expression of a protein having an equivalent molecular weight was observed.

The above results indicate that the addition of each of ASO1 and ASO2 induces exon skipping of exon 27 in a human WRN gene, though the levels are different, and thereby leading to expression of corresponding mutant-type protein.

### Example 2: Exon Skipping in iMSC Cells (1) (Morpholino Antisense Oligomer)

The WS-iMSC cells obtained in Production Example 4 were seeded in a 24-well plate. Every 72 hours from that point of time, ASO3 and ASO4 obtained in Production Example 1 were each added at a concentration of 10 µM to the cells using Endo-Porter reagent (manufactured by Gene Tools, LLC).

### Test Example 2-1: PDL Cell Growth Curve Analysis

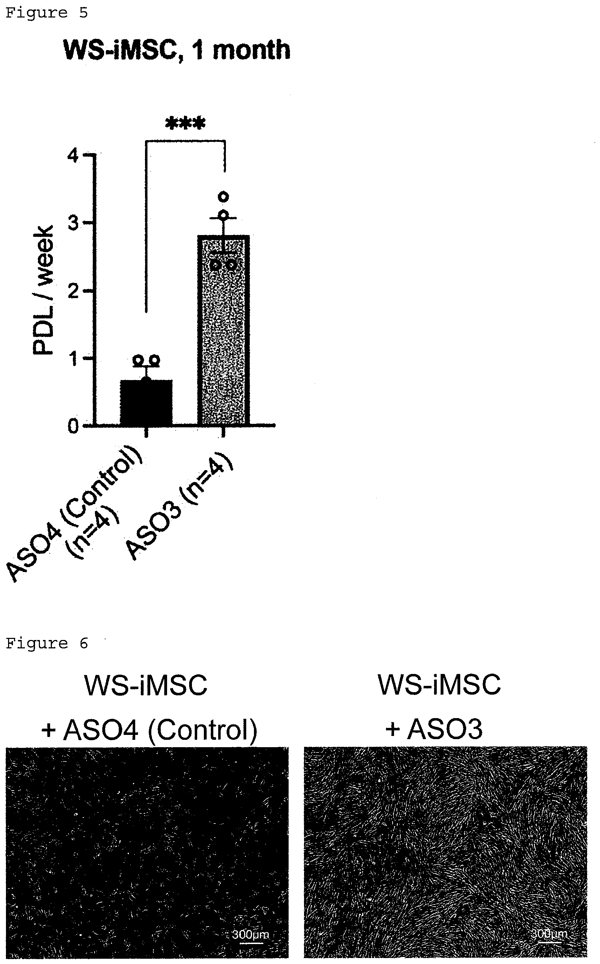

PDL cell growth curve analysis was performed on the WS-iMSC cells to which ASO3 and ASO4 were each added. The results are shown in Figures 4 and 5.

As shown in Figure 4, cell growth cessation was observed in the WS-iMSC cells to which the control ASO4 was added, at the time point of one month after the culture. On the other hand, significant continuation of cell growth was observed in the WS-iMSC cells to which ASO3 was added.

As shown in Figure 5, the value of change in cell growth potential, that is, population doubling level (PDL) per week after 30 days passage was 0.67 ± 0.20 (n=4) for the sample with ASO4 added. Contrary to this, the value of change in PDL for the sample with ASO3 added was 2.8 ± 0.26 (n=4, p value<0.006), and significant recovery of the cell growth potential was observed.

### Test Example 2-2: Cell Morphology Observation

Cell morphology observation was performed under the optical microscope on the WS-iMSC cells to which ASO3 and ASO4 were each added. The results are shown in Figure 6.

As shown in Figure 6, an increase in cells that indicates, for example, cell flattening and hypertrophy, and change in nuclear structure which are a typical phenotype of cellular senescence was observed in the WS-iMSC cells to which ASO4 was added. On the other hand, no increase in cells that indicates a phenotype of cellular senescence was observed in the WS-iMSC cells to which ASO3 was added.

### Test Example 2-3: SA-β-Gal Staining

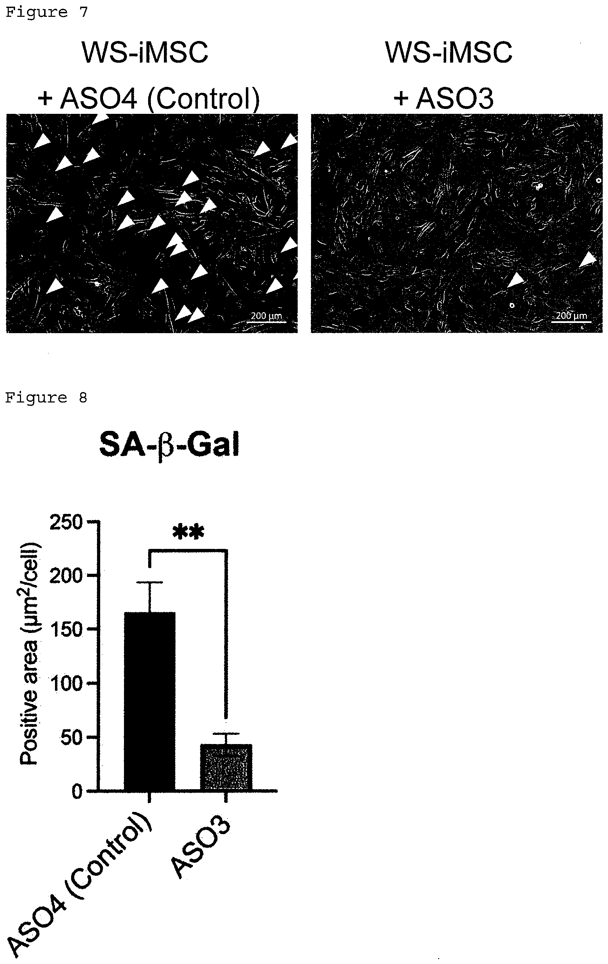

SA-β-Gal staining was performed on the WS-iMSC cells to which ASO3 and ASO4 were each added. The results are shown in Figures 7 and 8. Note that SA-β-Gal was found to be overexpressed in senescent cells and is an indicator of cellular senescence.

In SA-β-Gal staining images shown in Figure 7, a significant increase in SA-β-Gal positive cells were observed in the WS-iMSC cells to which ASO4 was added, indicating premature cellular senescence. On the other hand, no increase in SA-β-Gal positive cells was observed in the WS-iMSC cells to which ASO3 was added.

In the results of quantitative analysis of SA-β-Gal staining positive cells shown in Figure 8, a significant SA-P-Gal positive cells were observed in the WS-iMSC cells to which ASO4 was added, indicating premature cellular senescence. On the other hand, it was observed that the number of SA-β-Gal staining positive cells was significantly decreased in the WS-iMSC cells to which ASO3 was added.

Werner syndrome patients exhibit signs of premature aging in the whole body. Somatic cells derived from Werner syndrome patients also exhibit a phenotype of premature aging under in vitro culturing conditions. On the other hand, cells which initialized the cell differentiation do not exhibit a phenotype of aging.

The above results indicate that the phenotype of the progression of cellular senescence indicated by the somatic cells derived from Werner syndrome patients is decreased by the addition of ASO3.

### Test Example 2-4: Telomere Length Analysis

The telomere length was analyzed by a quantitative PCR method (qPCR) on the WS-iMSC cells to which ASO3 and ASO4 were each added.

The somatic cells derived from Werner syndrome patients are known to facilitate shortening of the telomere length associated with the progression of cellular senescence compared to healthy individuals. This is considered because the protein encoded by the WRN gene is a telomere-binding protein. Therefore, the telomere length is an indicator of functional activities of the WRN gene.

Specifically, a genome DNA was extracted from cell pellets of the population doubling level (PDL) of 20 using DNeasy Blood & Tissue Kit (manufactured by QIAGEN N.V.).

As a template, 20 ng of genome DNA, 0.1 mM each of a primer pair of teloF and teloR, or a primer pair of 36B4F and 36B4R shown in Table 5 below, as well as 2 X SYBR Green PCR Master Mix (manufactured by Thermo Fisher, Inc.) were added to a 96-well plate. PCR amplification response was performed using CFX Connect Real-Time System (manufactured by Bio-Rad Laboratories, Inc.) by the following cycles: priming at 95°C for 10 minutes followed by 40 cycles of 95°C for 15 seconds, and 60°C for 1 minute.

**[Table 5]**

| Table 5: Primer sequences for telomere length analysis | |
|---|---|
| teloF: | CGGTTTGTTTGGGTTTGGGTTTGGGTTTGGGTTTGGGTT (SEQ ID NO: 37) |
| teloR: | GGCTTGCCTTACCCTTACCCTTACCCTTACCCTTACCCT (SEQ ID NO: 38) |
| 36B4F: | CAGGAAGTGGGAAGGTGTAATCC (SEQ ID NO: 39) |
| 36B4R: | CCCATTCTATCATCAACGGGTACAA (SEQ ID NO: 40) |

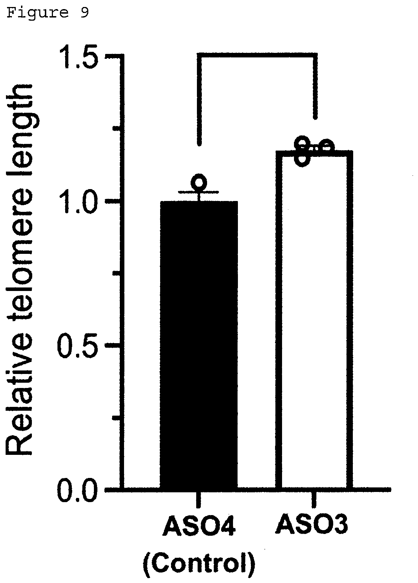

The telomere length was determined as a relative value by the delta-delta Ct method using the PCR amplified product of the primer pair of 36B4F and 36B4R as an internal control (Relative telomere length in the Figure). The results are shown in Figure 9.

As shown in Figure 9, the telomere length in the WS-iMSC cells with ASO3 added was significantly longer than that in the WS-iMSC cells with the control ASO4 added.

The above results indicate that the mutant-type protein induced in the WS-iMSC cells with ASO3 added keeps its functional activities.

### Test Example 2-4: Gene Expression Analysis

The gene expression analysis was performed by an RNA sequencing method on the WS-iMSC cells to which ASO3 and ASO4 were each added.

Total RNA was purified from PDL8 cell pellets using "PureLink RNA Mini Kit" (manufactured by Thermo Fisher, Inc.). Total RNA was used as a template, and a cDNA library was synthesized using NEBNext Ultra RNA Library Prep Kit (manufactured by New England Biolabs Inc., catalog No.: E7370S). Sequencing was performed using HiSeq1500 (manufactured by Illumina, Inc.) with 60 bp single-reads.

RNA-seq read mapping to the reference genome UCSC/hg19 was performed using TopHat. Annotation data was obtained from iGenomes (manufactured by Illumina, Inc.). For the quantification on the gene expression level, iDEP (see Ge SX, et al., BMC Bioinformatics. 2018; 19: 534) was used. Differentially expressed genes (DEG) analysis was performed under the conditions of a false discovery rate < 0.05, and twice the expression level served as cut-off, and the genes with a significant difference in the expression level between the WS-iMSC cells with ASO3 added and the WS-iMSC cells with ASO4 added were extracted.

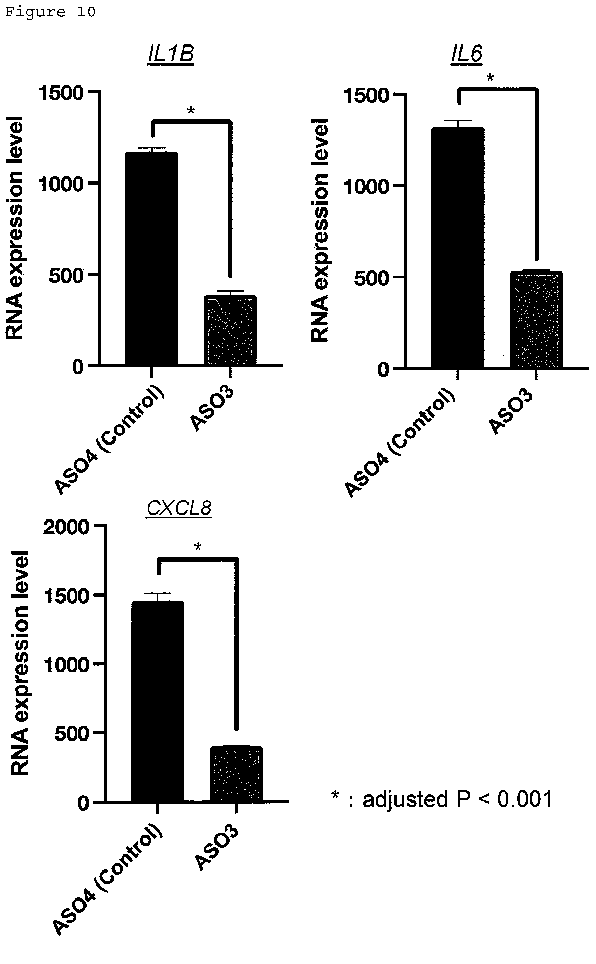

As the genes with a significant difference in the expression level, the aging-associated inflammatory genes, IL1B, IL6, CXCL8 and the like were extracted. Figure 10 shows the comparison of the expression level.

The expression level of the aging-associated inflammatory genes is known to facilitate in the somatic cells derived from Werner syndrome patients. On the other hand, as shown in Figure 10, the expression level of the aging-associated inflammatory genes, IL1B, IL6, and CXCL8 in the WS-iMSC cells with ASO3 added was significantly lower than that in the WS-iMSC cells with the control ASO4 added.

The above results indicate that the phenotype of the progression of cellular senescence exhibited by the somatic cells derived from Werner syndrome patients is decreased by the addition of ASO3.

### Example 3: Exon Skipping in iPS Cells (2) (Antisense Oligomer-Expressing Lentivirus)

The WS-iPS cells obtained in Production Example 2 and the WS-gene corrected iPS cells obtained in Production Example 3 were infected with the antisense oligo-expressing lentivirus using the concentrated virus solution obtained in Production Example 6, and the following experiments were performed. Accordingly, as is the case with the morpholino antisense oligomer, the exon skipping of exon 27 can also be confirmed to achieve in the case of using the antisense oligo-expressing lentivirus.

RT-PCR analysis in the same manner as in the above Test Example 1-1 is performed to confirm that the infection with the antisense oligo-expressing lentivirus can cause inducing the exon skipping of exon 27 in a human WRN gene.

Western blotting analysis in the same manner as in the above Test Example 1-2 is performed to confirm that the infection with the antisense oligo-expressing lentivirus causes inducing the exon skipping of exon 27 in a human WRN gene and leading to expression of corresponding mutant-type protein.

### Example 4: Exon Skipping in iMSC Cells (2) (Antisense Oligomer-Expressing Lentivirus)

The WS-iMSC cells obtained in Production Example 4 were infected with the antisense oligo-expressing lentivirus using the concentrated virus solution obtained in Production Example 6, and the following experiments are performed. Accordingly, as is the case of using the morpholino antisense oligomer, the effect of the exon skipping of exon 27 can also be confirmed in the case of using the antisense oligo-expressing lentivirus.

The PDL cell growth curve analysis, cell morphology observation, and SA-β-Gal staining are performed in the same manner as in Test Examples 2-1 to 2-3.

### Example 5: Exon Skipping in iPS Cells (3) (Morpholino Antisense Oligomer)

The WS-iPS cells obtained in Production Example 2 were seeded in a 24-well plate, and after 24 hours, ASO4 obtained in Production Example 1 as well as ASO5, ASO6, ASO7, ASO8, ASO9, ASO10, ASO11, ASO12, ASO13, ASO14, ASO15, and ASO16 which were obtained in Production Example 1 were each added at a concentration of 10 µM to the cells using Endo-Porter reagent (manufactured by Gene Tools, LLC) ("Endo-Porter (+)" in the Figure).

Further, ASO15 and ASO16 were each added at a concentration of 10 µM to the cells in the same manner except that Endo-Porter reagent was not used ("Endo-Porter (-)" in the Figure).

Thereafter, the culture was performed for 24 hours.

### Test Example 5-1: RT-PCR Analysis

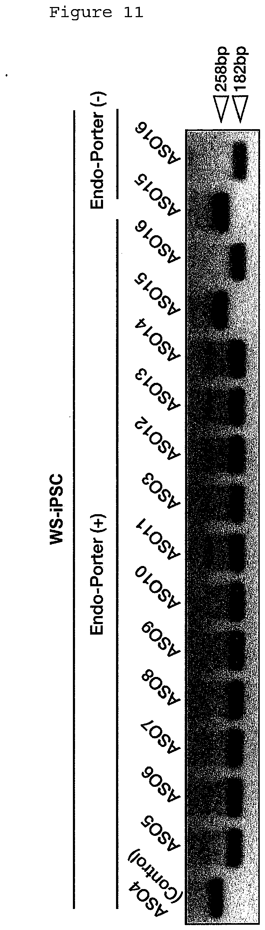

RT-PCR analysis was performed in the same manner as in the above Test Example 1-1. The results are shown in Figure 11.

As shown in Figure 11, a 258 bp mutant band with lack of exon 26 was observed in the WS-iPS cells to which the controls ASO4 and ASO15 were added.

On the other hand, a 182 bp band which was truncated due to lack of exon 27 as well as exon 26 in the human WRN gene by exon skipping was observed in the WS-iPS cells to which ASOS, ASO6, ASO7, ASO8, ASO9, ASO10, ASO11, ASO3, ASO12, ASO13, ASO14, or ASO16 was added.

Further, the 182 bp band was observed in the WS-iPS cells to which peptide-bound morpholino ASO16 was added, even in the case without using the Endo-Porter reagent.

The above results indicate that the addition of each of ASO5, ASO6, ASO7, ASO8, ASO9, ASO10, ASO11, ASO3, ASO12, ASO13, ASO14, and ASO16 can induce the exon skipping of exon 27 in the human WRN gene.

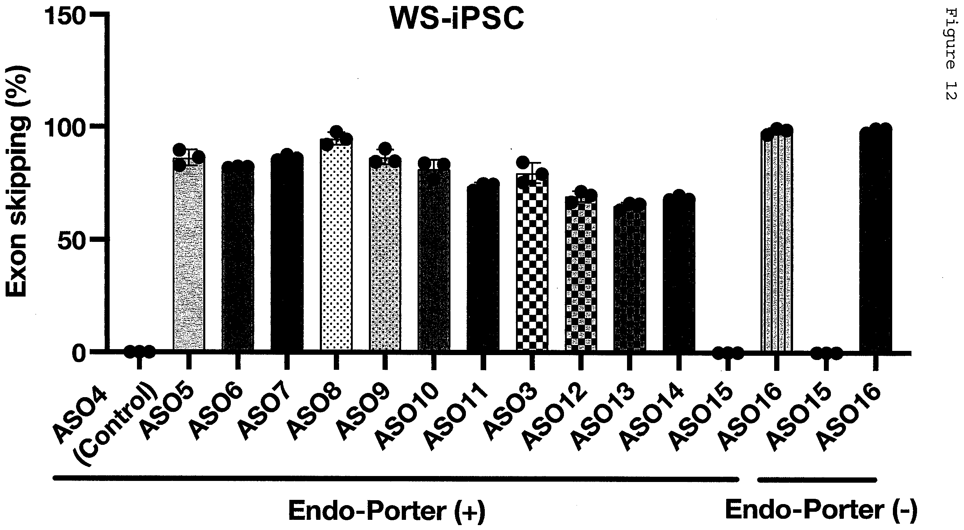

Further, the exon skipping efficiency was quantified in the same manner as in the above Test Example 1-2. The results are shown in Figure 12 and Table 6.

**[Table 6]**

| Table 6: Exon skipping efficiency | | |
|---|---|---|
| | Endo-Porter | Exon skipping efficiency (%) |
| AS05 | + | 86.3 ± 2.0 |
| AS06 | + | 82.2 ± 0.2 |
| AS07 | + | 86.3 ± 0.7 |
| AS08 | + | 94.9 + 1. 6 |
| AS09 | + | 86.7 ± 1.9 |
| AS010 | + | 81.5 ± 2.3 |
| AS011 | + | 73.9 ± 0.9 |
| AS03 | + | 79.8 ± 2.6 |
| AS012 | + | 69.5 ± 1.4 |
| AS013 | + | 65.3 ± 0.9 |
| AS014 | + | 68.7 ± 0.6 |
| AS016 | + | 98.5 ± 0.8 |
| AS016 | - | 98.9 ± 0.6 |

The above results indicate that the addition of each of ASO5, ASO6, ASO7, ASO8, ASO9, ASO10, ASO11, ASO3, ASO12, ASO13, ASO14, or ASO16 can induce exon skipping of exon 27 in the splicing of WRN gene in human WS patients, and as a result, the stop codon generated in exon 27 no longer functions and thereby leading to gene expression of mRNA up to the stop codon of normal WRN gene.

In addition, it was found that nearly 100% exon skipping efficiency was achieved when the peptide-bound morpholino ASO16 was added without using an induction reagent.

### Industrial Applicability

From the results of Examples, the antisense oligomer of the present invention is found to be capable of causing skipping of the 27th exon in a human WRN gene, and leading to expression of mutant-type protein whose functions are at least partially restored. That is, the antisense oligomer of the present invention can be used as an active ingredient of medicaments which can suppress premature aging symptoms of Werner syndrome.

## Claims

1. An antisense oligomer or a pharmaceutically acceptable salt thereof which consists of a base sequence complementary to the base sequence of the following (i) or (ii):
(i) a contiguous base sequence of 15 or more bases in the base sequence shown in SEQ ID NO: 1; or
(ii) a contiguous base sequence of 15 or more bases in the base sequence shown in SEQ ID NO: 1, having deletion, substitution, or insertion of one or more bases;
and which is capable of causing skipping of the 27th exon in a human WRN gene.

2. The antisense oligomer or the pharmaceutically acceptable salt thereof according to claim 1, wherein the contiguous base sequence in the base sequence (i) or the base sequence (ii) has a length of 20 bases or more and 40 bases or less.

3. The antisense oligomer or the pharmaceutically acceptable salt thereof according to claim 1, wherein the contiguous base sequence in the base sequence (i) or the base sequence (ii) comprises at least one of the base sequence shown in SEQ ID NO: 2 and the base sequence shown in SEQ ID NO: 3.

4. The antisense oligomer or the pharmaceutically acceptable salt thereof according to claim 3, wherein the contiguous base sequence in the base sequence (i) or the base sequence (ii) comprises at least one of the base sequence shown in SEQ ID NO: 4 and the base sequence shown in SEQ ID NO: 5.

5. The antisense oligomer or the pharmaceutically acceptable salt thereof according to claim 1, wherein the contiguous base sequence in the base sequence (i) or the base sequence (ii) consists of the base sequence shown in any of SEQ ID NOs: 6 to 15.

6. The antisense oligomer or the pharmaceutically acceptable salt thereof according to claim 1, wherein the antisense oligomer is an oligonucleotide, a morpholino oligomer, a peptide nucleic acid (PNA) oligomer, or a glycol nucleic acid (GNA) oligomer.

7. The antisense oligomer or the pharmaceutically acceptable salt thereof according to claim 1, wherein the antisense oligomer is a morpholino oligomer.

8. The antisense oligomer or the pharmaceutically acceptable salt thereof according to claim 7, wherein the morpholino oligomer is a phosphorodiamidate morpholino oligomer.

9. The antisense oligomer or the pharmaceutically acceptable salt thereof according to claim 6, wherein the oligonucleotide is an oligonucleotide comprising one or more selected from the group consisting of bridged nucleic acid (BNA) nucleotides.

10. The antisense oligomer or the pharmaceutically acceptable salt thereof according to claim 1, wherein at least one of the 5' end and the 3' end is modified.

11. A viral vector which expresses RNA capable of causing skipping of the 27th exon in a human WRN gene, said RNA consists of a base sequence complementary to the base sequence of the following (i) or (ii):
(i) a contiguous base sequence of 15 or more bases in the base sequence shown in SEQ ID NO: 1; or
(ii) a contiguous base sequence of 15 or more bases in the base sequence shown in SEQ ID NO: 1, having deletion, substitution, or insertion of one or more bases.

12. The viral vector according to claim 11, wherein the contiguous base sequence in the base sequence (i) or the base sequence (ii) comprises the base sequence shown in SEQ ID NO: 16.

13. A pharmaceutical composition for the treatment of Werner syndrome, comprising the antisense oligomer or the pharmaceutically acceptable salt thereof according to any one of claims 1 to 10, or the viral vector according to claim 11 or 12 as an active ingredient.

14. The pharmaceutical composition for the treatment of Werner syndrome according to claim 13, comprising two or more of the antisense oligomers or the pharmaceutically acceptable salts thereof as an active ingredient.

15. A pharmaceutical composition for inducing skipping of the 27th exon in a human WRN gene, comprising the antisense oligomer or the pharmaceutically acceptable salt thereof according to any one of claims 1 to 10, or the viral vector according to claim 11 or 12 as an active ingredient.
